Europäisches Patentamt

European Patent Office

Office européen des brevets

(11) Veröffentlichungsnummer: **0 298 261 B1**

# EUROPÄISCHE PATENTSCHRIFT

(45) Veröffentlichungstag der Patentschrift: **02.01.92**

(21) Anmeldenummer: **88109076.5**

(22) Anmeldetag: **07.06.88**

(51) Int. Cl.⁵: **C07C 255/24**, C07C 253/00, C07C 251/32

(54) Verfahren zur Herstellung von Salzen des Aminomalonsäuredinitrils.

(30) Priorität: **09.06.87 CH 2167/87**

(43) Veröffentlichungstag der Anmeldung:
**11.01.89 Patentblatt 89/02**

(45) Bekanntmachung des Hinweises auf die
Patenterteilung:
**02.01.92 Patentblatt 92/01**

(84) Benannte Vertragsstaaten:
**BE CH DE FR GB IT LI NL SE**

(56) Entgegenhaltungen:
**EP-A- 0 003 335**
**US-A- 3 670 007**

**JOURNAL OF THE CHEMICAL SOCIETY, PER-
KIN TRANSACTIONS I, Nr. 12, 1980, Seiten
2728-2731; K. KADIR et al.: "Purines, pyrimidines, and imidazoles. Part 56. Some aminoimidazolecarboxamidines and derived adenines"**

(73) Patentinhaber: **LONZA AG**

**Gampel/Wallis(CH)**

(72) Erfinder: **Mettler, Hanspeter, Dr.**
**Kirchweg 16**
**CH-Brig-Glis (Kanton Wallis)(CH)**

(74) Vertreter: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

EP 0 298 261 B1

**Beschreibung**

Die Erfindung betrifft ein Verfahren zur Herstellung von Salzen des Aminomalonsäuredinitrils. Es ist bekannt, Aminomalodinitril als p-Toluolsulfonat durch Reduktion von Oximinomalonsäuredinitril mit Aluminiumamalgam zu isolieren. Diese Herstellung ist jedoch langwierig und umständlich. Die Amalgamierung des Aluminiums bereitet Schwierigkeiten, da die Quecksilberschicht auf der Oberfläche des Aluminiums nicht gut haftet. Ausserdem bleibt beim Absaugen des Aluminiumhydroxids nach der Reduktion viel Produkt im Niederschlag zurück (J.P. Ferris, L.E. Orgel, J.Am.Chem.Soc. 87, 4976-7 (1965); J.P. Ferris, L.E. Orgel, J.Am.Chem.Soc. 88, 3829-31 (1966); J.P. Ferris US-Patent 3 670 007 (1972), C.A. 77, 100866v (1972); J.P. Ferris, R.A. Sanchez, R.W. Mancuso, Org.Synth. 48, 1-3). J.P. Ferris beschreibt in seinem Patent auch Reduktionen mit Zink und Natriumdithionit, wobei er sich jedoch auf den qualitativen Nachweis von Aminomalonsäuredinitril in der Reaktionslösung beschränkt.

Es ist auch ein Verfahren bekannt, bei dem man Oximinomalonsäuredinitril in Gegenwart von Raneykatalysatoren als Reduktionsmittel bei Wasserstoffdrücken von 5 bis 7 bar (4 bis 6 atü) und Temperaturen von 10 bis 80°C in Tetrahydrofuran als Lösungsmittel umsetzt und das Aminomalonsäuredinitril durch Behandlung mit p-Toluolsulfonsäure aus der Reaktionslösung ausfällt und als Tosylat isoliert oder in Acetanhydrid als Lösungsmittel zum Aminomalonsäuredinitril umsetzt und dieses als Acetylaminomalonsäuredinitril ausfällt und isoliert (EP 3335). Nach diesen Verfahren werden Ausbeuten von 27 bis 49% erzielt. Aufgabe der Erfindung ist es, ein verbessertes Verfahren zur Verfügung zu stellen, nach dem wesentlich höhere Ausbeuten resultieren.

Die Aufgabe wurde durch ein Verfahren, wie es in Patentanspruch 1 gekennzeichnet ist, gelöst.

Die Nitrosierung des Malonsäuredinitrils wird erfindungsgemäss bei einem pH von 3,8 bis 4,2, vorzugsweise 4, in einem Gemisch $H_2O/H_2SO_4$, zweckmässig mit Natriumnitrit durchgeführt. Das Gemisch $H_2O/H_2SO_4$ besteht vorzugsweise aus 15 bis 30 Mol Wasser und 0,02 bis 0,05 Mol Schwefelsäure, bezogen auf 1 Mol Malonsäuredinitril. Die vorteilhaft zur Anwendung gelangende Temperatur liegt bei 15 bis 25°C.

Das nach der Nitrosierung anfallende Reaktionsgemisch wird vorzugsweise nach Zusatz von 0,5 bis 1,0 Mol Schwefelsäure pro Mol eingesetztes Malonsäuredinitril extrahiert.

Die Extraktion des Nitrosierungsproduktes kann mit Methylenchlorid, Diethylether oder Ethylacetat durchgeführt werden. Die Hydrierung erfolgt in polaren organischen Lösungsmitteln, beispielsweise in Ethanol, Ether, Tetrahydrofuran oder Ethylacetat. Die Lösungsmittel können alleine oder in Kombination mit organischen Säuren wie Essigsäure, Ameisensäure, Toluolsulfonsäure oder anorganischen Säuren wie Salzsäure, Phosphorsäure, Schwefelsäure verwendet werden.

Nach einer bevorzugten Arbeitsweise wird zur Extraktion und Hydrierung das selbe Lösungsmittel angewendet.

Für diese bevorzugte Arbeitsweise eignet sich besonders Ethylacetat.

Vorteilhaft werden zur Extraktion 15 bis 30 Mol, vorzugsweise 20 Mol Ethylacetat pro Mol eingesetztes Malonsäuredinitril angewendet.

Die das Oximierungsprodukt enthaltende Extraktionslösung kann mit Essigsäure auf ein Molverhältnis Essigsäure/Ethylacetat von 2 bis 15 zu 1 eingestellt und dann der Hydrierung zugeführt werden.

Dabei hat es sich als zweckmässig gezeigt, einen Teil des Ethylacetates abzuziehen und dann die benötigte Essigsäure (als 100%ige) zuzusetzen.

Der Hydrierungskatalysator ist ein Edelmetallkatalysator. Solche sind bespielsweise Platin, Palladium oder Rhodium, die auf einem Träger, vorzugsweise auf Kohlenstoff, aufgebracht sind. Es kann aber auch $PtO_2$ zur Anwendung kommen. Vorzugsweise wird Platin in einer Konzentration von I bis 10%, vorteilhaft von 5% aufgebracht auf Kohlenstoff, verwendet. In einer zweckmässigen Ausführungsform wendet man 8 bis 30 g, vorzugsweise 15 g, Katalysator pro Mol eingesetztes Malonsäuredinitril an. Nach der Hydrierung wird das Reaktionsgemisch zweckmässigerweise filtriert.

Dem Aminomalonsäuredinitril enthaltenden Filtrat wird darauf die entsprechende Säure, zweckmässig Methansulfonsäure, p-Toluolsulfonsäure oder konzentrierte Salzsäure zugesetzt. In der Regel kann nach teilweisem Einengen der Reaktionslösung und mittels Zugabe eines apolaren organischen Lösungsmittels, wie z.B. Toluol, das entsprechende Säuresalz ausgefällt und darauf isoliert werden.

Gegebenenfalls kann das Säuresalz einer zusätzlichen Reinigung durch Umkristallisation unterzogen werden.

Beispiel 1

Herstellung von Aminomalonsäuredinitril-p-toluolsulfonat

Zu 13,2 g (0,200 Mol) Malonsäuredinitril (MDN) in 60 g Wasser setzte man bei 20°C innert 30 Minuten 13,8 g (0,200 Mol) Natriumnitrit in 40 g Wasser zu. Der pH-Wert der Lösung wurde dabei durch Zugabe von Schwefelsäure konstant auf dem Wert 4 gehalten. Die Lösung wurde während 4 Stunden bei 20˚ C gerührt, mit 70 g 20%iger Schwefelsäure versetzt und mit 4 x 90 g Ethylacetat extrahiert. Man trocknete die organische Phase während 2 Stunden über Natriumsulfat und engte auf 150 g ein. Diese, das Hydroximinomalonsäuredinitril enthaltende Phase mischte man mit 250 g Essigsäure und hydrierte bei 10 bar Wasserstoffdruck und Raumtemperatur über 3,0 g Pt/C (5%). Das Reaktionsgemisch wurde filtriert, das Filtrat mit 38,0 g (0,200 Mol) p-Toluolsulfonsäure versetzt, auf total 280 g eingeengt, mit 600 g Toluol vermischt und 12 Stunden bei 4˚ C stehengelassen. Das ausgefallene Aminomalonsäuredinitril-p-toluolsulfonat wurde abfiltriert, mit Toluol gewaschen und getrocknet.

Die Ausbeute war: 44,93 g (74,9% der Theorie).

Smp: 163,5 bis 163,8˚ C.

Nach Umkristallisation aus Ethanol wurde ein Produkt mit einem Smp. von 167,0 bis 168,0˚ C erhalten.

Beispiel 2

Herstellung von Aminomalonsäuredinitrilmethansulfonat

Eine Lösung von 4,76 g (0,050 Mol) Hydroximinomalonsäuredinitril in 38 g Ethylacetat (hergestellt wie in Beispiel 1 beschrieben) wurde mit 60 g Essigsäure vermischt und bei 10 bar Wasserstoffdruck und Raumtemperatur über 0,8 g Pt/C (5%) hydriert. Das Reaktionsgemisch wurde filtriert, das Filtrat mit 4,81 g (0,050 Mol) Methansulfonsäure versetzt, auf 74 g eingeengt, mit 175 g Toluol vermischt und 12 Stunden bei 4˚ C stehengelassen. Das ausgefallene Aminomalonsäuredinitrilmethansulfonat wurde abfiltriert, mit Toluol gewaschen und getrocknet. Man erhielt 8,43 g Produkt in einer Ausbeute von 83,3% (bez. Hydroximinomalonsäuredinitril).

Durch Umkristallisation aus Ethanol isolierte man ein Produkt mit einem Smp. von 149 bis 151˚ C.

NMR: (300 MHz, DMSO-d[6])
7,85 (s, 3H), 6,10 (s, 1H), 2,49 (s, 3H)

Beispiel 3

Herstellung von Aminomalonsäuredinitrilhydrochlorid

Eine Lösung von 4,76 g (0,050 Mol) Hydroximinomalonsäuredinitril in 38 g Ethylacetat (hergestellt wie in Beispiel 1 beschrieben) wurde mit 60 g Essigsäure vermischt und bei 10 bar Wasserstoffdruck und Raumtemperatur über 0,8 g Pt/C (5%) hydriert. Das Reaktionsgemisch wurde filtriert, das Filtrat mit 7,33 g (0,075 Mol) konzentrierter Salzsäure versetzt, auf 65 g eingeengt, mit 175 g Toluol versetzt und 12 Stunden bei 4˚ C stehengelassen. Das ausgefallene Aminomalonsäuredinitrilhydrochlorid wurde abfiltriert, mit Toluol gewaschen und getrocknet. Man erhielt 4,10 g Produkt in einer Ausbeute von 63,4% (bez. Hydroximinomalonsäuredinitril).

Smp.: Zersetzung ab etwa 160˚ C.
NMR: (300 MHz, DMSO-d[6])
8,25 (s, 3H), 6,34 (s, 1H)

**Patentansprüche**

1. Verfahren zur Herstellung von Salzen des Aminomalonsäuredinitrils, bei dem man Oximinomalonsäuredinitril hydriert, und das Aminomalonsauredinitril als Säuresalz isoliert, dadurch gekennzeichnet, dass man Malonsäuredinitril bei einem pH von 3,8 bis 4,2 in einem Gemisch aus $H_2O/H_2SO_4$ nitrosiert, das Nitrosierungsprodukt mit einem mit Wasser nicht mischbaren Lösungsmittel extrahiert, die Hydrierung in Gegenwart eines Edelmetallkatalysators bei Wasserstoffdrücken von 1 bis 40 bar und Temperaturen von 10 bis 50˚ C in einem polaren Lösungsmittel durchführt, und das Aminomalonsäuredinitril durch Behandlung mit einer Säure aus der Reaktionslösung ausfällt und als Säuresalz isoliert.

2. Verfahren nach Patentanspruch 1, dadurch gekennzeichnet, dass man die Nitrosierung mit Natriumnitrit in einem Gemisch aus 15 bis 30 Mol Wasser und 0,02 bis 0,05 Mol $H_2SO_4$, bezogen auf 1 Mol Malonsäuredinitril, durchführt.

3. Verfahren nach Patentansprüchen 1 bzw.2, dadurch gekennzeichnet, dass zur Extraktion und Hydrierung das gleiche Lösungsmittel verwendet wird.

4. Verfahren nach Patentansprüchen 1, 2 bzw.3, dadurch gekennzeichnet, dass als Extraktionsmittel und als Lösungsmittel für die Hydrierung Ethylacetat verwendet wird.

5. Verfahren nach Patentansprüchen 1, 2, 3 bzw. 4, dadurch gekennzeichnet, dass die Hydrierung in einem Gemisch aus Ethylacetat/Essigsäure durchgeführt wird.

6. Verfahren nach Patentansprüchen 1, 2, 3, 4 bzw.5, dadurch gekennzeichnet, dass man die Hydrierung in einem Gemisch aus Essigsäure und Ethylacetat in einem Molverhältnis von 2 bis 15 Molen Essigsäure zu 1 Mol Ethylacetat durchführt.

7. Verfahren nach Patentansprüchen 1, 2, 3, 4, 5 bzw. 6, dadurch gekennzeichnet, dass man als Edelmetallkatalysator Platin auf einem Kohlenstoffträger verwendet.

8. Verfahren nach Patentansprüchen 1, 2, 3, 4, 5, 6 bzw. 7, dadurch gekennzeichnet, dass man als Säuren Methansulfonsäure, p-Toluolsulfonsäure oder Salzsäure einsetzt.

## Claims

1. Process for the preparation of salts of amino malonic dinitrile in which oximino malonic dinitrile is hydrogenated and the amino malonic dinitrile is isolated as the acid salt, characterized in that malonic dinitrile is nitrosated at a pH value of 3.8 to 4.2 in a mixture of $H_2O/H_2SO_4$, the nitrosation product is extracted with a water-immiscible solvent, the hydrogenation is carried out in the presence of a noble metal catalyst at hydrogen pressures of 1 to 40 bar and at temperatures of 10 to 50 °C in a polar solvent, and the amino malonic dinitrile is precipitated from the reaction solution by treatment with an acid and isolated as acid salt.

2. Process according to patent claim 1, characterized in that the nitrosation is carried out with sodium nitrite in a mixture of 15 to 30 moles of water and 0.02 to 0.05 moles of $H_2SO_4$, based on 1 mole of malonic dinitrile.

3. Process according to patent claims 1 and 2 resp., characterized in that the same solvent is used in the extraction and in the hydrogenation.

4. Process according to patent claims 1, 2 and 3 resp., characterized in that as extracting agent and as solvent for the hydrogenation ethyl acetate is used.

5. Process according to patent claims 1, 2, 3 and 4 resp., characterized in that the hydrogenation is carried out in a mixture of ethyl acetate/acetic acid.

6. Process according to patent claims 1, 2, 3, 4 and 5 resp., characterized in that the hydrogenation is carried out in a mixture of acetic acid and ethyl acetate in a molar ratio of 2 to 15 moles of acetic acid to 1 mole of ethyl acetate.

7. Process according to patent claims 1, 2, 3, 4, 5 and 6 resp., characterized in that platinum on a carbon carrier is used as noble metal catalyst.

8. Process according to patent claims 1, 2, 3, 4, 5, 6 and 7 resp., characterized in that as acids methane sulfonic acid, p-toluene sulfonic acid or hydrochloric acid are used.

## Revendications

1. Procédé de préparation de sels du dinitrile de l'acide aminomalonique, dans lequel on hydrogène le dinitrile de l'acide oximinomalonique et on isole le dinitrile de l'acide aminomalonique sous forme de sel d'acide, caractérisé en ce que l'on nitrose le dinitrile de l'acide malonique à un pH de 3,8 à 4,2 dans un mélange de $H_2O/H_2SO_4$, on extrait le produit de nitrosation avec un solvant immiscible à l'eau, on réalise l'hydrogénation en présence d'un catalyseur de métal noble sous des pressions d'hydrogène de 1 à 40 bar et à des températures de 10 à 50 °C dans un solvant polaire et on précipite de la solution réactionnelle le dinitrile de l'acide aminomalonique par traitement avec un acide et on l'isole sous forme de sel d'acide.

2. Procédé selon la revendication 1, caractérisé en ce que l'on réalise la nitrosation avec le nitrite de sodium dans un mélange de 15 à 30 moles d'eau et 0,02 à 0,05 mole de $H_2SO_4$ pour 1 mole de dinitrile de l'acide malonique.

3. Procédé selon les revendications 1 ou 2, caractérisé en ce que l'on utilise le même solvant pour l'extraction et l'hydrogénation.

4. Procédé selon les revendications 1, 2 ou 3, caractérisé en ce que l'on utilise l'acétate d'éthyle comme agent d'extraction et comme solvant pour l'hydrogénation.

5. Procédé selon les revendications 1, 2, 3 ou 4, caractérisé en ce que l'hydrogénation est réalisée dans un mélange acétate d'éthyle/acide acétique.

6. Procédé selon les revendications 1, 2, 3, 4 ou 5, caractérisé en ce que l'on réalise l'hydrogénation dans un mélange d'acide acétique et d'acétate d'éthyle dans un rapport molaire de 2 à 15 moles d'acide acétique par mole d'acétate d'éthyle.

7. Procédé selon les revendications 1, 2, 3, 4, 5 ou 6, caractérisé en ce que l'on utilise le platine sur un support de carbone comme catalyseur de métal noble.

8. Procédé selon les revendications 1, 2, 3, 4, 5, 6 ou 7, caractérisé en ce que l'on utilise comme acides l'acide méthanesulfonique, l'acide p-toluènesulfonique ou l'acide chlorhydrique.